# EUROPEAN PATENT APPLICATION

(11) **EP 3 295 865 A1**
(43) Date of publication of application: **21.03.2018**
(21) Application number: 16791945.5
(22) Date of filing: 25.03.2016
(51) Int. Cl.: A61B 5/00

(54) **METHOD FOR DETECTING, IN NONINVASIVE IN-VIVO MANNER, SKIN DAMAGE INDUCED BY ULTRAVIOLET LIGHT, AND DETECTION DEVICE THEREOF**

(30) Priority: 08.05.2015 CN 201510231662
(71) Applicant: Shanghai Jiao Tong University, Shanghai 200240 (CN)
(72) Inventor: YING, Weihai, Shanghai 200030 (CN); ZHANG, Mingchao, Shanghai 200030 (CN); HE, Hao, Shanghai 200030 (CN); ZHANG, Jie, Shanghai 200030 (CN)
(74) Representative: Hervouet-Malbec, Sylvie
(86) International application number: PCT/CN2016/077399
(87) International publication number: WO 2016/180095

(57) **Abstract**

The current invention has provided a method for detecting, in a noninvasive in-vivo manner, UV-induced skin damage and a detection device thereof. After irradiated by ultraviolet light, the autofluorescence is generated at the wavelength ranging from 490 nm to 640 nm under the skin, following the excitement of laser having a wavelength ranging from 440 nm to 510 nm, wherein the changes of the autofluorescence intensity at the wavelength of 490-640 nm is positively correlated with the skin damages.

## Description

### Technical Field

The invention relates to a method and device for detecting autofluorescence of body. Specifically,, the invention relates to a *in vivo* non-invasive method for detecting UV-induced skin damage and the detection devices thereof.

### Background

The ultraviolet (UV) rays from the sunlight is the main cause for UV-induced damage of human skin. UV radiation can generate multiple pathological alterations of the skin, including skin redness, peeling, inflammation, fester and various skin diseases and the like. UV can also produce a large amount of reactive oxygen species that can cause the DNA damage of subcutaneous cells and accelerate the aging of skin. In fact, UV irradiation causes 90% of aging of human skin, and the most severe disease induced by UV is skin cancer. It has been demonstrated that 90% of skin cancer is caused by UV from the sunlight. Skin cancer patients account for 40 percent of all cancer patients, and there are 3 million new skin cancer patients in the world every year. The Skin Cancer Foundation also indicates that one fifth of American people will suffer from skin cancer at a certain stage in their life. With the development of human industrialization, the ozone sphere that is responsible for blocking UV is continuously thinned. Experts have predicted that the thickness of ozone sphere is decreased by each 1%, the UV intensity on the earth would increase by 2%. Therefore, the detection of UV-induced damage is socially economically and clinically beneficial to improve the health of skin and avoid severe skin diseases.

In China, due to such problems as huge population pressure and the difficulties in diagnosis and hospitalization, the attention on UV-induced skin damage has not been sufficient. Many patients with skin damage will only go to see a doctor when they have severe skin damage, which has led to the increases in the number of patients with skin damage or skin cancer in China recently. Therefore, it is of great significance to develop efficient methods to detect UV-induced skin damage and to develop low cost and portable devices, which would significantly enhance human being's capacity in detection, diagnosis and treatment of skin diseases, especially skin cancer.

Currently, the clinical diagnosis of UV-induced skin damage is based on the visual inspection of dermatologists, which highly depends on doctors' experience. The doctors can provide evaluation only for severe skin damage, and they are not able to provide early diagnosis of UV-induced skin damage, especially skin cancer. The current devices for detecting skin damage can only provide very rough estimations on skin conditions. The principle for the devices is as follows: skin is irradiated to UV lamp, then the devices is used to detect the light that is reflecting from the skin. Since the light absorption coefficients of the normal skin tissue and the damaged region are different, a rough estimation of the content of melanin, water and fat subcutaneously can be carried out by detecting the light that is reflecting or scattering from the skin. Because the propagation of laser ray in tissues is very sensitive to scattering, and according to Rayleigh Scattering and Mie Scattering Theory, the amount of photon scattering is inversely proportional to the fourth power of wavelength, for UV that is an ultra-short wavelength light, the scattered interference can have a significant effect when UV spreads in skin tissues. Meanwhile, the absorption of UV is very strong in normal cells. Therefore, this kind of reflection imaging will be strongly interfered by many factors. Therefore, the accuracy and repeatability are very low. When this technique is applied on the same person, for different tissues or for the same tissue but different muscle conditions, the difference in possible results may have been comparable to that caused by UV damage. Therefore, such devices have disadvantages such as low resolution and low accuracy. In addition, it cannot be used to detect UV-induced early-stage skin damage, or provide guidance for early detection and prevention of skin damage and incidence of skin diseases. Therefore, this kind of devices has little clinical application. Especially in dermatology clinics, only qualitative evaluation can usually be performed on the UV sensitivity of the skin by using a light experiment: A non-visible area of the skin, such as the skin of the back, is irradiated to UV irradiation. The UV sensitivity of the skin is then evaluated on the basis of the size of erythema. This method is not accurate, and it not directly detect skin damage.

### Content of Invention

The inventor finds that, after the skin is irradiated to UV irradiation, the autofluorescence with the wavelength of 490-640 nm is generated subcutaneously, following the irradiation of the skin to the excitation light at the excitation wavelength of 440-510 nm. The change of the autofluorescence intensity at the wavelength of 490-640 nm is in direct proportion relation with the skin damage. Meanwhile, X-rays cannot induce the similar increases of the autofluorescence at these wavelengths. Therefore, our invention is based on this novel discovery, which can overcome the technological difficulties in the prior art so as to establish not only a novel detection method for precise and non-invasive prediction of UV-induced skin damage, but also a corresponding detection devices for UV-induced skin damage.

In one aspect, the present invention relates to a method for in vivo, non-invasive detection of UV-induced skin damage, comprising the following steps:
(1) Within 72 hours after the skin of the subjects is irradiated by a light source containing certain doses of UV irradiation, the skin of the subjects is irradiated by a light source containing certain doses of UV irradiation is placed under excitation light at the wavelength raging from 440 nm to 510 nm so as to induce subcutaneous autofluorescence, wherein, the dosage of UV = Power of UV × Irradiation time;
(2) Detecting the autofluorescence intensity of the skin at the wavelength in the range between 490-640 nm emitted from the locations between the stratum corneum and the dermis layer of the skin of the subjects irradiated by the light source containing UV;
(3) In according with the method of the Step (2), detecting the autofluorescence intensity of the skin of the subjects that has not been irradiated to light source containing UV ;
(4) Comparing the autofluorescence intensity of the skin irradiated to light source containing UV and that of the skin that has not been irradiated to light source containing UV, determining the change rate of the autofluorescence intensity, which is induced by the UV irradiation;
(5) Predicting the heath condition of the skin of the subjects, according to the change rate of the autofluorescence intensity which is induced by the UV irradiation.

In the detection method of the present invention, preferably, the detection is performed within 48 hours after the skin of the subject is irradiated to UV irradiation; more preferably, the detection is performed within 24 hours.

In the detection method of the present invention, the method to induce the autofluorescence by excitation light includes at least one of the method that applies normal, continuous light output, or the method that modulates excitation light by electric modulation, or the method that uses pulse laser.

In the detection method of the present invention, the wavelength of the excitation light preferably ranges from 460 nm to 500 nm, and more preferably, the wavelength ranges from 485 nm to 490 nm.

In the detection method of the present invention, the wavelength for the detected autofluorescence preferably ranges from 500 nm to 550 nm; and more preferably, the wavelength ranges from 500 nm to 530 nm.

In another aspect, the present invention relates to an in vivo and noninvasive device to detect ultraviolet-induced skin damage comprising an excitation light source, an optical transmission system, and an imaging system.

In the foresaid detection device of the present invention, the excitation light source comprises at least one of the a single-frequency laser, or a Narrowband light source, or a Broadband light source, all of which can emit light having a wavelength that ranges from 440 nm to 510 nm; optionally includes at least one piece of band-pass filter; the filter is used to filter the polychromatic light emitted from the excitation light source into monochromatic light of the desired wavelength.

In the foresaid detection device of the present invention, the optics transmission system is used to transmit the excitation light to the skin of the subject and to transmit auto fluorescence from the skin of the subject to the imaging system; wherein, the excitation light and the autofluorescence together transmit in a part of the optics transmission system, and is separated by the optical transmission system; the optical transmission system includes a dichroic mirror for separating the excitation light and the autoflorescence, a pair of scanning galvanometer for modulating the position of facula, and a pair of Conjugate lens for modulating the imaging plane of the excitation light.

According to some embodiments of the present invention, in the foregoing optical transmission system of detection device in the present invention,
the excitation light and the autofluorescence are transmitted reversely along the main light path of the optical transmission system, the dichroscope, the scanning galvanometers and the conjugate lens are arrayed along the main axis;
one of the excitation light source and the imaging system being located on the main axis and the other is on a side axis perpendicular to the main axis;
the dichroic mirror being located at an intersecting position of the main axis and the side axis, then the excitation light and the autofluorescence are separated into a right-angled relationship, so that only the autofluorescence enters the imaging system.

In the foresaid detection device of the present invention, the imaging system includes the components for detecting fluorescence images, which is capable of imaging the light having a wavelength ranging from 490 nm to 640 nm, and is capable of calculating the intensity of the light. According to some embodiments of the present invention, the imaging system includes at least one of a photomultiplier tube (PMT), an Avalanche Photodiodes (APD), a photodiode (PD), a CCD, or a CMOS photodetector.

The present invention can precisely predict the extent of UV-induced skin damage, based on our finding that the UV-induced subcutaneous autofluorescence that occurs at the early stage after the UV irradiation, is directly correlated with the skin damage that occurs at the late stage. This invention has established basis for prevention and early treatment of UV-induced skin damage, which can greatly reduce the incidence of the UV-induced skin diseases. At the same time, the detection device provided by the present invention, as small-sized detection devices for fluorescence images, can be used not only in the high-end, precise medical diagnosis and research of skin damage which involves fluorescence reconstruction, but also for the low-end medical diagnosis of skin damage and home use for detection of skin damage. These devices have the significant advantages including high effectiveness/cost ratios, high precision and wideness of the applications.

Figures Legends:

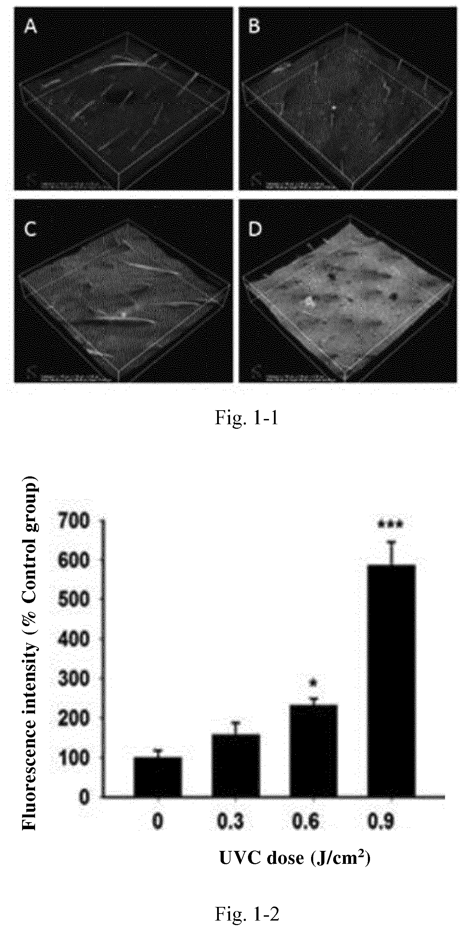

Fig. 1-1 shows an increase in the subcutaneous autofluorescence at 24 hours after the UVC irradiation.
Fig. 1-2 shows the histogram of the increase in the subcutaneous autofluorescence at 24 hours after the UVC irradiation.
Fig. 2 shows the H&E staining of C57 mice at 24 hours at 5 days after UVC injury.
Fig. 3 shows an increase in the subcutaneous autofluorescence at 24 hours after the UVC irradiation.
Fig. 4 shows representative graph of the H&E staining of C57 mice at 5 days after the UVC irradiation.
Fig. 5 shows the histogram of the subcutaneous autofluorescence and keratinization at 5 days after the UVC irradiation.
Fig. 6 shows the contrast graph of the TUNEL staining and epidermal apoptosis in C57 mice at 5 days after the UVC irradiation.
Fig. 7 shows an increase in the subcutaneous autofluorescence at 24 hours after the UVB irradiation.
Fig. 8 shows the representative H&E staining graph of the ear area of C57 mice at 24 hours after UVB irradiation.
Fig. 9 shows the representative H&E staining histogram of the ear area of C57 mice at 24 hours after UVB irradiation.
Fig. 10 shows the representative H&E staining graph of epidermal area of C57 mice at 24 hours after UVB irradiation.
Fig. 11 shows the representative H&E staining histogram of the epidermal area of C57 mice at 24 hours after UVB irradiation.
Fig. 12 shows the representative H&E staining graph of the ear area of C57 mice at 5 days after UVB irradiation.
Fig. 13 shows the representative H&E staining histogram of the ear area of C57 mice at 5 days after UVB irradiation.
Fig. 14 shows the representative H&E staining graph of epidermal area of C57 mice at 5 days of UVB irradiation.
Fig. 15. shows the representative H&E staining histogram of epidermal area of C57 mice at 5 days of UVB irradiation.
Fig. 16-1 shows the invariant graph of autofluorescence at 8 or 24 hours after synchrotron radiation X-ray irradiation.
Fig. 16-2 shows the histogram of subcutaneous autofluorescence at 8 or 24 hours after synchrotron radiation X-ray irradiation.
Fig. 17 shows the representative graph of the subcutaneous autofluorescence of nude mice at 24 hours after UVC irradiation.
Fig. 18 shows the representative graph of the subcutaneous autofluorescence of ICR mice at 24 hours after UVC irradiation.
Fig. 19 shows the representative graph of the subcutaneous autofluorescence of mice at 6 hours after irradiation by a solar simulator.
Fig. 20 shows the representative graph of the subcutaneous autofluorescence of human index fingers after UV irradiation.
Fig. 21 shows the schematic structure of the detection device for in vivo and non-invasive detection of UV-induced skin damage.

### Detailed description

The invention will now be described further by the way of specific description.

Unless otherwise defined, all technical and scientific terms used herein have the same meaning as commonly understood by one of ordinary skill in the art to which this invention pertains.

The term "ultraviolet light (UV light)", as used in the present invention means the electromagnetic radiation having a wavelength in the range of 100 to 400 nm, which includes naturally occurring light, such as the UVA, UVB and UVC in the solar light, as well as the man-made light at this range of wavelength. The ultraviolet light referred to in the present invention includes continuous electromagnetic radiation, pulsed electromagnetic radiation, and modulated electromagnetic radiation, the strength thereof is not particularly defined.

The term of "UV-induced skin damage" as used in this invention means the damage including UV-induced tanning of skin, sunburn, skin photoaging, allergic reactions of skin, and skin cancer.

The term of "autofluorescence" as used in this invention is the light with longer wavelength than that of excitation light, generated in the cellular endogenous molecule by the excitation light while it exits the excited state, after the biomolecule enters excited state after the molecule absorbs the energy of the excitation light during the process that the molecule is irradiated to the excitation light of certain wavelength.

The term of "excitation light" as used in this invention is the light that can induce autofluorescence by exciting certain biomolecules, which has shorter wavelength that that of autofluorescence.

The relationship between the changes of UV-induced autofluorescence of skin and the extent of skin damage

The inventors have conducted a large number of experiments, which have established the relationship between the changes of UV-induced autofluorescence of skin and the extent of skin damage.

According to the present invention, male C57 mice, male ICR mice and male nude mice are used in this invention. The weight of the mice used for UVC irradiation ranges from 15 g to 25g, while the weight of the mice used for UVB irradiation ranges from 18 g to 25g. After the mice are anesthetized, 1: 1 solvent of glycerol to water is smeared on the skin of the mice, followed by the corresponding UV light. After the UV irradiation, the mice are housed in experimental animal room with conditions of 22-24°C, with a 12 hour bright / dark cycle. Water and food are freely available to the mice.

One day later, laser confocal microscope is used for noninvasive imaging of the skin irradiated with UV light. The mice are sacrificed after one or five days, and the skin tissues are collected. The skin tissues are tested using the H&E staining (hematoxylin and eosin staining) and TUNEL staining (in situ terminal marker) . The skin tissues of the mice are imaged with laser confocal microscope, where the excitation wavelength of the laser confocal microscope is 500-550 nm.

The storage of skin tissue: After the skin tissues are collected, the skin tissues are soaked into 4% paraformaldehyde, which is then used for paraffin sectioning. The rest of the skin tissues is wrapped in aluminum-foil paper, frozen by liquid nitrogen, kept in a -80 °C refrigerator for long-term preservation.

Paraffin section of skin tissues: Soak the skin tissues in 4% paraformaldehyde for 24 hours. As the approach for performing paraffin section, the tissues are soaked sequentially into running water, distilled water, different gradients of ethanol, dimethylbenzene and paraffin.

The H&E staining of paraffin sections: Soak the paraffin section into dimethylbenzene to perform dewaxing, then soak the paraffin sections sequentially in different gradients of ethanol and distilled water, and then stain the paraffin sections in hematoxylin (10 minutes). Wash the paraffin sections by running water for 30 minutes, then soak sequentially the paraffin sections into distilled water for 30 seconds and 95% ethanol for 10 seconds. Then stain the paraffin sections in eosin for 30 seconds. After washing the paraffin sections twice by 70% ethanol, soak sequentially the paraffin sections in different gradients of ethanol and dimethylbenzene, and then seal the paraffin sections by neutral resins.

Imaging and quantifications of the stained paraffin sections: Take photos of the stained paraffin sections under microscope, and then quantify the skin keratinized thickness, epidermal thickness.

Statistical analysis: All data are shown in the form of Mean + Standard Deviations. All of data are evaluated by one-way analysis of variance. It is considered statistically significant, if the P value is less than 0.05.

The results of the animal experiments are shown as follows:

Figure 1: One day after the skin is irradiated by UVC, subcutaneous spontaneous fluorescence with the UV radiation dose is dose-dependent increased significantly..

Figure 1-1 from Figure 1: After the UVC irradiation in the ear of C57 at different times, Twenty-four hours after the ear of C57 mice is irradiated to various doses of UVC, spontaneous fluorescence intensity is increased with the irradiation time.

Group A,B,C,D are Control groups, the irradiation dose is 0.3 J/cm², 0.6 J/cm², and 0.9 J/cm² respectively.

Figure 1-2 from Figure 1: Quantifications of the subcutaneous autofluorescence to the ear. * represents that the P value is less than 0.05; *** represents that the P value is less than 0.001.

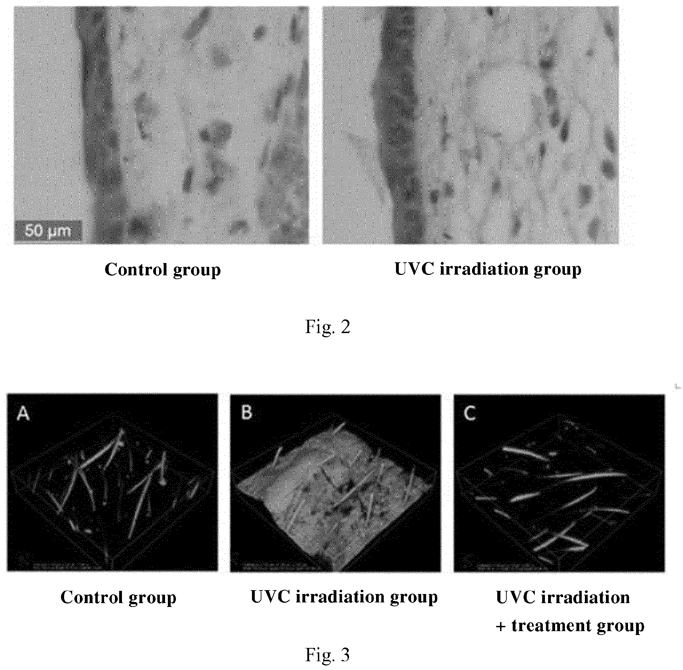

Figure 2: One day after UV irradiation, H&E staining assay did not show observable skin damage.

Figure 3: Five days after the UVC irradiation, H & E staining shows that the amount of autofluorescence detected at 1 day after the UVC irradiation is positively correlated with the skin damage at five days after the UVC irradiation. Group A, B, and C show the subcutaneous autofluorescence of control group, the group with 40-min UVC irradiation, and the group with the solution containing 50% water and 50% glycerine.

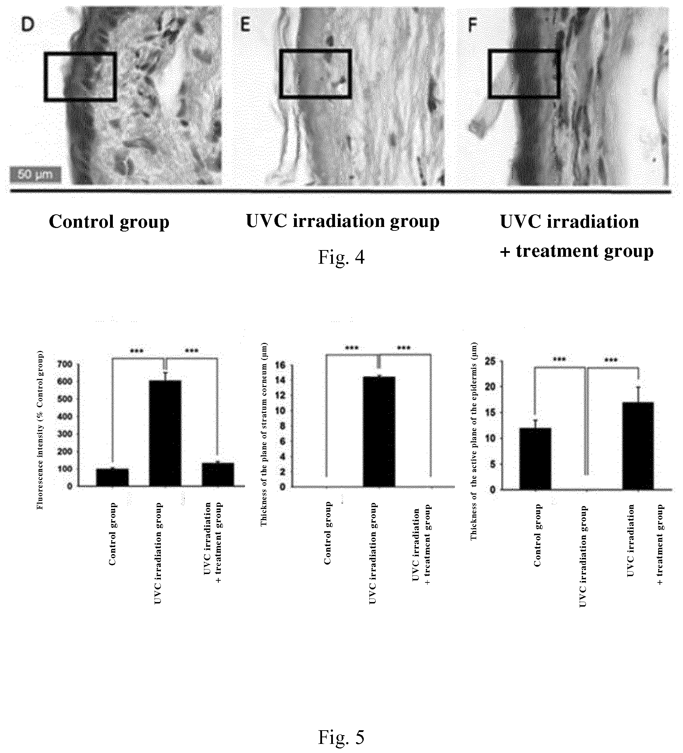

Figure 4: Group D, E, and F are the graphs of representative H&E staining of the skin tissues. The section shown in the box is the epidermis.

Figure 5: Group G is the quantification graph of the intensity of the autofluorescence; Group H is the quantification graph of the active plane of the epidermis; and the Group I is the quantification graph of the plane of stratum corneum.

As shown in Figure 5, the subcutaneous autofluorescence is determined at one day after UVC irradiation, and the skin damage is assessed at one day after UVC irradiation by H&E staining. By analyzing these results, the inventors find strong positive correlation between the autofluorescence intensity at one day after UVC irradiation and the skin damage at five days after UVC irradiation. The inventors also find negative correlation between the autofluorescence intensity and the thickness of active epidermis other than the stratum corneum (the loss of active epidermis is an index of skin damage). The inventor further finds positive correlation between the autofluorescence intensity and the thickness of epidermal keratinization (increased cornification is also an index of skin damage). Therefore, this invention reveals publicly that the enhancement of subcutaneous autofluorescence can be used as an index for predicting future skin damage.

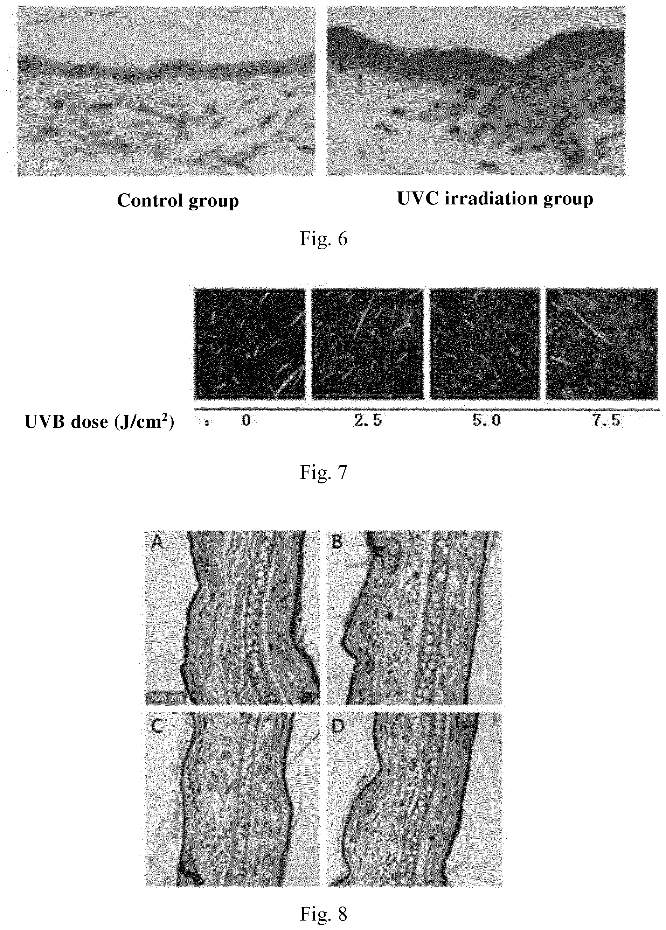

Figure 6: TUNEL staining of the skin. The dark part in this graph is the positive signal of TUNNEL staining, which represents cellular apoptosis. At five days after UVC irradiation, there is positive correlation between the autofluorescence intensity and the TUNEL signal (TUNEL signal is one of the index of skin damage).

Figure 7: At one day after UVB irradiation, the subcutaneous autofluorescence intensity shows UVB dose-dependent increases. The increased fluorescence is in the form of circular fluorescent signal. At Twenty-four hours after irradiation to various doses of UVB, the subcutaneous autofluorescence intensity shows UVB dose-dependent increases.

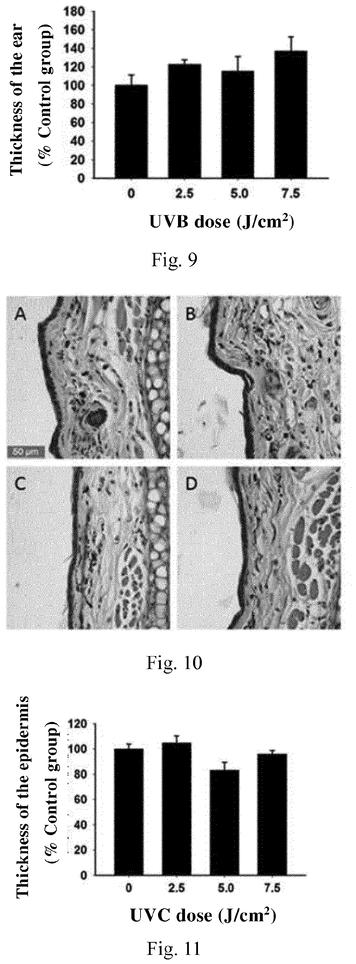

Figure 8 and Figure 9: The 100 × graphs of the H&E staining of the skin tissue, at 24 hours after UVB irradiation. The graph does not show any obvious change of the thickness of active epidermis, suggesting that there is no obvious skin damage.

Figure 10 and Figure 11: The 20 × graphs of the H&E staining of the skin tissue, at 24 hours after UVB irradiation. The graph does not show any obvious change of the thickness of active epidermis, suggesting that there is no obvious skin damage.

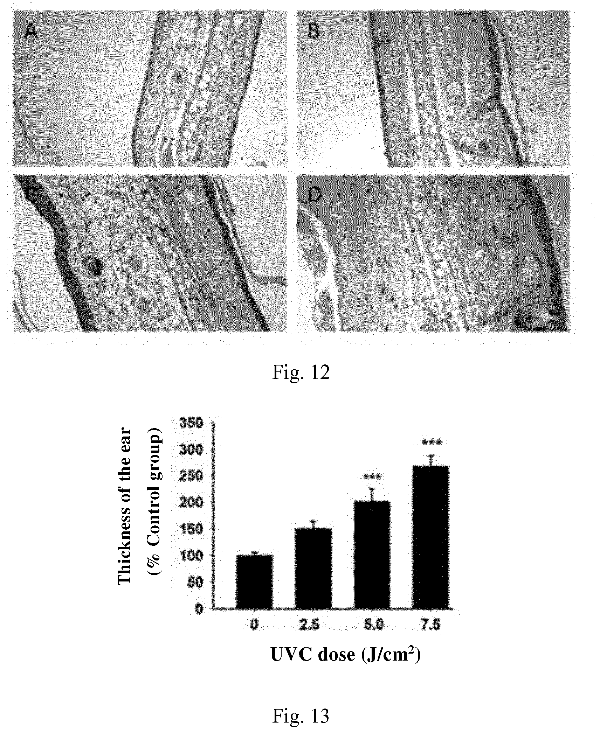

Figure 12 and figure 13: The H&E staining shows that there is a positive correlation between the intensity of the subcutaneous autofluorescence at one day after UVB irradiation and the skin damage at five days after UVB irradiation. Graph A, B, C, D are the representative graph of H&E staining of 5 days after UVB irradiation for the groups of Control, 2.5 J/cm², 5.0 J/cm², and 7.5 J/cm² respectively. Graph E is the quantification graph of the thickness of the ear. The subcutaneous autofluorescence intensity was determined at one day after UVB irradiation, and the skin damage was determined at five days after UVB irradiation by H&E staining. The quantifications show a positive correlation between the autofluorescence and the skin damage. There is a positive correlation between the autofluorescence and the thickness of the ears.

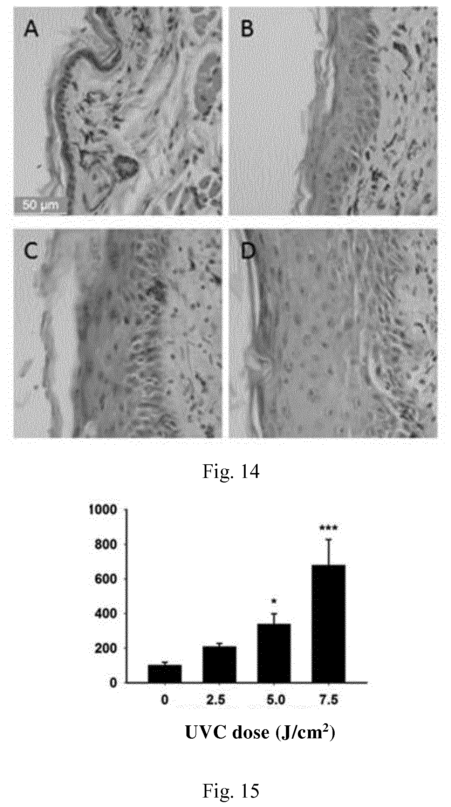

Figure 14 and figure 15: The H&E staining shows that there is a positive correlation between the intensity of the subcutaneous autofluorescence intensity at one day after UVB irradiation and the skin damage at five days after UVB irradiation. Graph A, B, C, D are the representative graph of H&E staining of 5 days after UVB irradiation for the groups of Control, 2.5 J/cm², 5.0 J/cm², and 7.5 J/cm² respectively. Graph E is the quantification graph of the thickness of epidermis. The subcutaneous autofluorescence intensity was determined at one day after UVB irradiation, and the skin damage was determined at five days after UVB irradiation by H&E staining. The quantifications show a positive correlation between the autofluorescence and the thickness of epidermis. These results suggest that the increased green fluorescence can be used as a novel biomarker to predict the skin damage in the future.

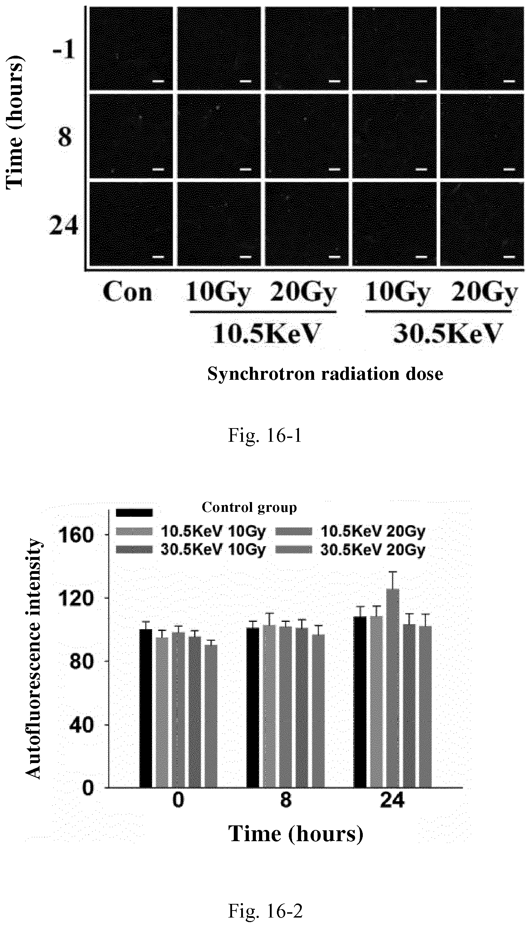

Figure 16-1 and Figure 16-2: At Eight hours or one day after X-ray irradiation, the subcutaneous autofluorescence is not significantly be changed with the increasing doses of X-ray.

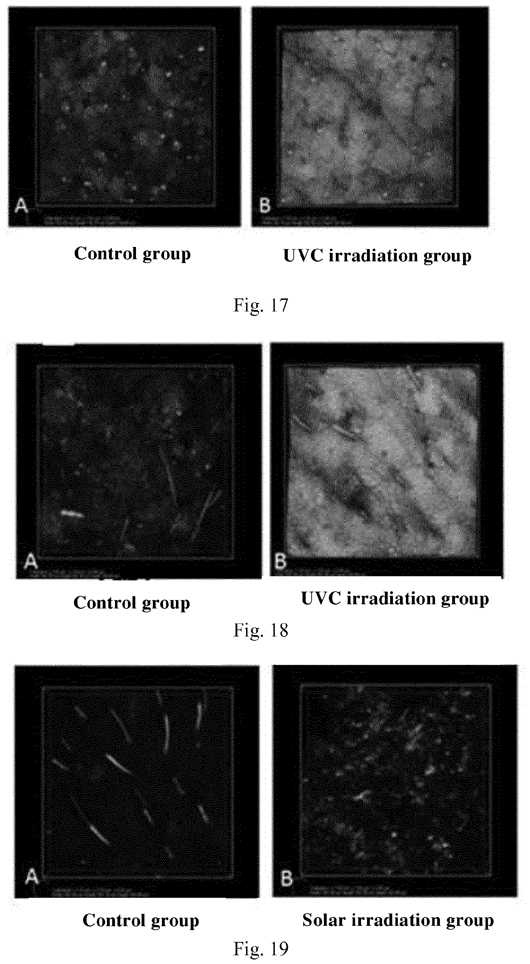

Figure 17: The change of the subcutaneous autofluorescence, after UVC irradiation of nude mice skin. As shown in the graph, the UVC irradiation also led to increased subcutaneous autofluorescence of nude mice skin.

Figure 18: The change of the subcutaneous autofluorescence, after UVC irradiation of ICR mice skin. As shown in the graph, the UVC irradiation also led to increased subcutaneous autofluorescence of UVC irradiation of nude mice skin.

Figure 19: The change of autofluorescence, after the human skin is irradiated to sunlight. As shown in the graph, the sunlight irradiation lead to increased autofluorescence in the C57 skin.

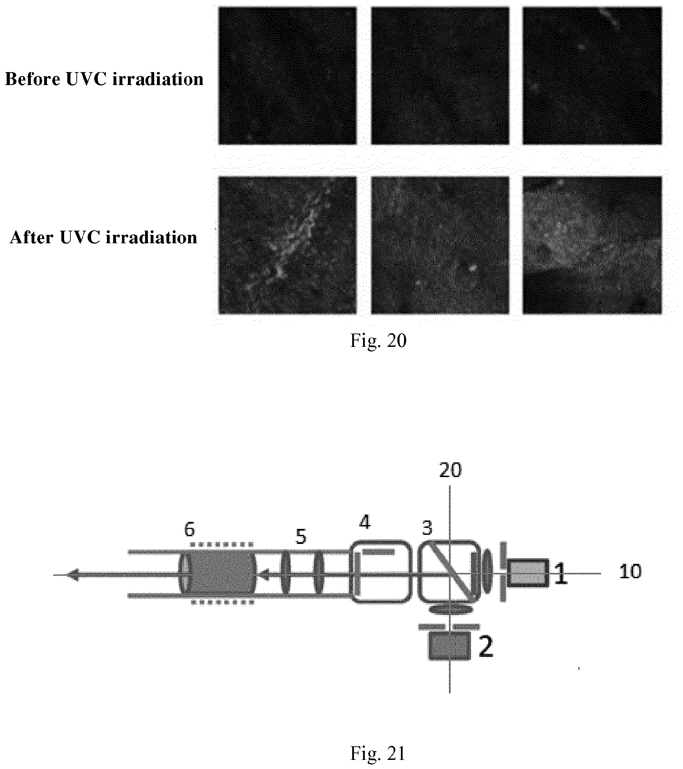

Figure 20: The change of autofluorescence, after the human skin is irradiated to UVC. As shown in the graph, the UVC irradiation lead to increased autofluorescence in the human skin.

The experimental results stated above have collectively shown that, after the skin is irradiated to UV irradiation (UVB and UVC), the autofluorescence at the wavelength of 490-640 nm is generated subcutaneously, following the irradiation of the skin to the excitation light at the excitation wavelength of 440-510 nm. The changes of the autofluorescence intensity at the wavelength of 490-640 nm is positively correlated with the skin damage. In contrast, X-rays cannot induce the similar increases in the autofluorescence at these wavelengths. Therefore, the subcutaneous autofluorescence can become a biomarker for predicting the skin damage.

Our invention is established on the basis of this finding,
providing a method for *in vivo,* non-invasive detection of UV-induced skin damage, comprising the following steps:
(1) Within 72 hours after the skin of the subjects is irradiated by a light source containing a certain dose of UV irradiation, the skin of the subjects is irradiated by a light source containing certain doses of UV irradiation is placed under excitation light at the wavelength ranging from 440 nm to 510 nm so as to induce subcutaneous autofluorescence;
(2) Detecting the autofluorescence intensity of the skin at the wavelength in the range between 490-640 nm emitted from the locations between the stratum corneum and the dermis layer of the skin of the subjects irradiated by the light source containing UV;
(3) In according with the method of the Step (2), detecting the auto fluorescence intensity of the skin of the subjects that has not been irradiated to light source containing UV;
(4) Comparing the autofluorescence intensity of the skin irradiated to light source containing UV and that of the skin that has not been irradiated to light source containing UV, determining the change rate of the autofluorescence intensity, which is induced by the UV irradiation;
(5) Predicting the heath condition of the skin of the subjects, according to the change rate of the autofluorescence intensity which is induced by the UV irradiation.

In the present invention, the method to induce the autofluorescence by excitation light includes at least one of the method that applies normal, continuous light output, or the method that modulates excitation light by electric modulation, or the method that uses pulse laser.

In the detection method of the present invention, preferably, the detection is conducted within 48 hours after the light source irradiation; and more preferably, the detection is conducted within 24 hours after the light source irradiation.

In the detection method of the present invention, preferably, the wavelength of excitation light ranges from 460 nm to 500 nm; and more preferably, the wavelength of excitation light ranges from 485 nm to 490 nm.

In the detection method of the present invention, preferably, the wavelength of autofluorescence ranges from 500 nm to 550 nm; and more preferably, the wavelength of autofluorescence ranges from 505 nm to 530 nm.

The method for in vivo, non-invasive detection of UV-induced skin damage, as described in this invention, is to predict the extent of the UV-induced skin damage of the subjects, based on the principle that the change of the subcutaneous autofluorescence is proportional with the extent of the skin damage.

In addition, our invention has introduced a detection device for in vivo, non-invasive detection of UV-induced skin damage. The detection device can be a small-sized fluorescence imaging device, comprising an excitation light source, an optical transmission system, and an imaging system.

In the detection device of this invention, the excitation light source comprises at least one of the a single-frequency laser, or a Narrowband light source, or a Broadband light source, all of which can emit light having a wavelength that ranges from 440 nm to 510 nm; the excitation light source also includes at least one piece of band-pass filter; the band-pass filter is used for filtering the polychromatic light from the source of excitation light into the monochromatic light at the wavelength required for the detection.

In the detection device of this invention,
the optics transmission system is used to transmit the excitation light to the skin of the subject and to transmit autofluorescence from the skin of the subject to the imaging system; wherein, the excitation light and the autofluorescence together transmit in a part of the optics transmission system, and is separated by the optical transmission system;
the optical transmission system includes a dichroic mirror for separating the excitation light and the autoflorescence, a pair of scanning galvanometer for modulating the position of facula, and a pair of Conjugate lens for modulating the illumination thickness of the excitation light.

In the optical transmission system of the detection device of this invention, the excitation light and the autofluorescence are transmitted reversely along the main light path of the optical transmission system, the dichroscope, the scanning galvanometers and the conjugate lens are arrayed along the main axis; one of the excitation light source and the imaging system being located on the main axis and the other is on a side axis perpendicular to the main axis; the dichroic mirror being located at an intersecting position of the main axis and the side axis, then the excitation light and the autofluorescence are separated into a right-angled relationship, so that only the autofluorescence enters the imaging system.

In the detection device of this invention, the imaging system includes the components for detecting fluorescence images, which is capable of imaging the light having a wavelength ranging from 490 nm to 640 nm, and is capable of calculating the intensity of the light. In some embodiments, the fluorescence imaging system includes at least one of a photomultiplier tube (PMT), a Avalanche Photodiodes (APD), a photodiode (PD), a CCD, or a CMOS photodetector.

As shown in Figure 21, a schematic diagram of one example to accomplish the inventive detection equipment is designed. According to Figure 21 as reference, the inventive, intravital, non-invasive equipment, for detection of UV-induced skin damage, is then explained.

As shown in Figure 21, the main elements are arranged along the main axis 10, including PMT detector 1, dichroscope 2, a pair of scanning galvanometers 4, a pair of conjugate lens 5 and objective lens 6. The side axis 20 is perpendicular to main axis 10. Some elements are arranged in the side axis 20, including a 488-nm semiconductor laser unit 2, and dichroscope 3. Obviously, the dichroscope 3 is located at the intersection position of the main axis 10 and side axis 20.

The laser light emitted from the laser unit 2 is used as the excitation light passes through the small hole and is collimated by a short focal length lens, and then reaches into the dichroscope 3. Then the excitation light will be reflected by the dichroscope 3 into the scanning galvanometer 4. Subsequently, it penetrates through the pair of conjugate lens 5 and is focused by the objective lens 6. Wherein a pair of scanning galvanometers 4 may be used to adjust the position of the light spot on a plane perpendicular to the main axis 10 and a pair of conjugate lenses 5 may be used to adjust the focus position of the excitation light on the main aixis 10, i.e., the irradiation depth of the excitation light.

The auto-fluorescence signal is excited and then collected by the same objective lens 6. The signal then propagates back through the pair of conjugate lens 5 and reflected by scanning galvanometer 4. It then passes through dichroscope 3 and a filter straightly and is then focused by a lens into a pinhole. After that, the PMT detector 1 can detect the autofluorescence signal.

It should be noted that according to the spectrum design of the dichroscope 3, the position of detector 1 and laser unit 2 is replaceable with each other. The positional interchangeable detection device is also included in the scope of the present invention..

As a miniaturized fluorescence detection imaging apparatus, the control system of scanning galvanometer 3, PMT detector 1 and laser unit 2 can be installed properly in any proper spaces. The size of the whole system could be controlled in the range of 30 cm × 10 cm × 10 cm.

In addition to the above-described elements, this autofluorescence detection design can be also accomplished by other optical elements, such as, a light source that can emit light including the blue band. For example, a 473 nm laser unit, a narrow band blue LED, a mercury lamp with color filter can all work as the light source. Other optical units can also be used in this invention, such as silver mirror, convex lens, aspherical mirror, filters, optical gratings (with aperture), CCD ,CMOS, etc.

It will be understood by those skilled in the art that the detection apparatus of the present invention can be implemented using other methods and elements, based on the principles of the testing apparatus of the present invention. For example, the dichroscope can be replaced by a beam splitter and corresponding filters to separate the excitation light and the auto-fluorescence signal. The filters can also be replaced by optical gratings or any other elements that can disperse the spectrum spatially. As long as the excitation light and auto-fluorescence can be split spatially, the autofluorescence can be detected. The optical path design can be different from the foregoing examples accordingly. Besides, in this invention, taking accounts of cost and the accuracy, the scanning galvanometer for point-by-point scanning, and the conjugate lens for the propagation control of light, can both be omitted. These modified and / or simplified detection devices are also within the scope of the present invention.

When using this inventive intravital, non-invasive detection equipment to predict ultraviolet light induced skin damage, the skin of experimental subject is firstly excited by the excitation light. The auto-fluorescence from the skin tissue is then collected by the objective lens and coupled into the photomultiplier or the imaging sensor. The intensity and the wavelength of the auto-fluorescence will be finally detected by the system.

It will be appreciated that the practice of the present invention for noninvasive detection of ultraviolet light-induced skin damage is not dependent on the testing apparatus of the present invention. The method of in vivo non-invasive detection of ultraviolet light-induced skin damage of the present invention can be carried out using any apparatus capable of emitting excitation light and capable of detecting the autofluorecence.

It will be apparent to those skilled in the art that, although for purposes of illustration, specific embodiments of the invention are described herein, various modifications may be made thereto without departing from the spirit and scope of the invention. Accordingly, the specific embodiments and examples of the present invention should not be construed as limiting the scope of the invention. The invention is limited only by the appended claims. All documents cited in this application are hereby incorporated by reference in their entirety.

## Claims

1. A method for *in vivo*, non-invasive detection of UV-induced skin damage, comprising the following steps:
(1) Within 72 hours after the skin of the subjects is irradiated by a light source containing a certain dose of UV irradiation, the skin of the subjects is irradiated by a light source containing certain doses of UV irradiation is placed under excitation light at the wavelength ranging from 440 nm to 510 nm so as to induce subcutaneous autofluorescence;
(2) Detecting the autofluorescence intensity of the skin at the wavelength in the range between 490-640 nm emitted from the locations between the stratum corneum and the dermis layer of the skin of the subjects irradiated by the light source containing UV;
(3) In according with the method of the Step (2), detecting the autofluorescence intensity of the skin of the subjects that has not been irradiated to light source containing UV;
(4) Comparing the autofluorescence intensity of the skin irradiated to light source containing UV and that of the skin that has not been irradiated to light source containing UV, determining the change rate of the autofluorescence intensity, which is induced by the UV irradiation;
(5) Predicting the heath condition of the skin of the subjects, according to the change rate of the autofluorescence intensity which is induced by the UV irradiation.

2. The method according to claim 1, **characterized in that**:
the method to induce the autofluorescence by excitation light includes at least one of the method that applies normal, continuous light output, or the method that modulates excitation light by electric modulation, or the method that uses pulse laser.

3. The method according to claim 1, **characterized in that**:
The detection is conducted within 48 hours after the light source irradiation.

4. The method according to claim 3, **characterized in that**:
The detection is conducted within 24 hours after the light source irradiation.

5. The method according to claim 1, **characterized in that**:
The wavelength of excitation light ranges from 460 nm to 500 nm.

6. The method according to claim 5, **characterized in that**:
The wavelength of excitation light ranges from 485 nm to 490 nm.

7. The method according to claim 1, **characterized in that**:
The wavelength of autofluorescence ranges from 500 nm to 550 nm.

8. The method according to claim 7, **characterized in that**:
The wavelength of autofluorescence ranges from 505 nm to 530 nm.

9. A detection device for *in vivo,* non-invasive detection of UV-induced skin damage, comprising an excitation light source, an opticial transmission system, and an imaging system, **characterized in that**:
the excitation light source comprises at least one of the a single-frequency laser, or a Narrowband light source, or a Broadband light source, all of which can emit light having a wavelength that ranges from 440 nm to 510 nm;
the optics transmission system is used to transmit the excitation light to the skin of the subject and to transmit autofluorescence from the skin of the subject to the imaging system; wherein, the excitation light and the autofluorescence together transmit in a part of the optics transmission system, and is separated by the optical transmission system;
the imaging system includes the components for detecting fluorescence images, which is capable of imaging the light having a wavelength ranging from 490 nm to 640 nm, and is capable of calculating the intensity of the light.

10. The detection device according to claim 9, **characterized in that**:
the excitation light source also includes at least one piece of band-pass filter;
the optical transmission system includes a dichroic mirror for separating the excitation light and the autoflorescence, a pair of scanning galvanometer for modulating the position of facula, and a pair of Conjugate lens for modulating the illumination thickness of the excitation light.

11. The detection device according to claim 10, **characterized in that**:
the excitation light and the autofluorescence are transmitted reversely along the main light path of the optical transmission system, the dichroscope, the scanning galvanometers and the conjugate lens are arrayed along the main axis;
one of the excitation light source and the imaging system being located on the main axis and the other is on a side axis perpendicular to the main axis;
the dichroic mirror being located at an intersecting position of the main axis and the side axis, then the excitation light and the autofluorescence are separated into a right-angled relationship, so that only the autofluorescence enters the imaging system.

12. The detection device according to claim 9, **characterized in that**: the imaging system includes at least one of a photomultiplier tube (PMT), a Avalanche Photodiodes (APD), a photodiode (PD), a CCD, or a CMOS photodetector.
